(19) Europäisches Patentamt
European Patent Office
Office européen des brevets

(11) **EP 4 578 414 A1**

(12) **EUROPEAN PATENT APPLICATION**
published in accordance with Art. 153(4) EPC

(43) Date of publication:
**02.07.2025 Bulletin 2025/27**

(21) Application number: **22963627.9**

(22) Date of filing: **02.12.2022**

(51) International Patent Classification (IPC):
$A61B\ 18/14^{(2006.01)}$    $A61B\ 18/20^{(2006.01)}$
$A61B\ 18/02^{(2006.01)}$    $A61N\ 1/40^{(2006.01)}$
$A61N\ 1/06^{(2006.01)}$    $A61N\ 1/05^{(2006.01)}$
$A61N\ 1/36^{(2006.01)}$    $A61N\ 7/02^{(2006.01)}$

(52) Cooperative Patent Classification (CPC):
A61B 18/02; A61B 18/14; A61B 18/20; A61N 1/05;
A61N 1/06; A61N 1/36; A61N 1/40; A61N 7/02

(86) International application number:
**PCT/KR2022/019470**

(87) International publication number:
**WO 2024/090666 (02.05.2024 Gazette 2024/18)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **25.10.2022 KR 20220138127**

(71) Applicant: **Deepqure Inc.**
**Seoul 06243 (KR)**

(72) Inventors:
• **YOON, Young Il**
**Seoul 06243 (KR)**
• **HONG, Suk Ki**
**Seoul 06243 (KR)**
• **BACH, Du Jin**
**Seoul 06243 (KR)**
• **JO, Seok Hyeon**
**Seoul 06243 (KR)**
• **KIM, Kee Wan**
**Seoul 06243 (KR)**
• **PARK, Chan Ho**
**Seoul 06243 (KR)**
• **PARK, Jong Sun**
**Seoul 06243 (KR)**

(74) Representative: **Manitz Finsterwald**
**Patent- und Rechtsanwaltspartnerschaft mbB**
**Martin-Greif-Strasse 1**
**80336 München (DE)**

(54) **ELECTRODE APPARATUS FOR BLOCKING OR CONTROLLING NERVES IN BODY**

(57)    An electrode apparatus for nerve denervation or modulation in vivo includes: a main body including a shaft; and an electrode unit formed to protrude from one end of the shaft, configured to denervate or modulate at least a part of nerves on a tube in a body, and including a base layer and an electrode layer disposed on the base layer. The electrode unit includes: a first electrode disposed along a longitudinal direction of one side of the base layer; an electrode layer including a second electrode disposed along a longitudinal direction of the other side of the base layer; a current sensor unit formed in a region between the first electrode and the second electrode and configured to measure a current generated from the first electrode or the second electrode; a plurality of temperature sensor units spaced apart from each other at a predetermined interval between the first electrode and the second electrode and configured to measure a temperature; and a controller configured to calculate a resistance based on the current measured by the current sensor unit and calculate a diameter of the tube in the body based on the calculated resistance.

*FIG. 1*

## Description

### TECHNICAL FIELD

The present disclosure relates to an electrode apparatus for nerve denervation or modulation in body.

### BACKGROUND

Denervation is a surgical procedure intended to control an abnormally overactive autonomic nervous system by damaging specific nerves. For example, renal denervation can treat hypertension and heart diseases by damaging renal sympathetic nerves directed to the kidney, and pulmonary denervation can treat lung diseases by damaging parasympathetic nerves directed to the lung.

Nerves usually enclose the outer walls of tubes, such as blood vessels, bronchial tubes, etc., and it may be necessary to enclose the outer walls of tubes to measure signals from the nerves or transmit electrical impulses or various energies to the nerves to damage or destroy the nerves.

For example, when a surgical procedure is performed on the renal artery, the main renal artery which is a procedure target has a diameter of from 5 mm to 7 mm, and the accessory renal artery having a diameter of from 1 mm to 2 mm may also be a procedure target. Also, the artery with distributed nerves varies in size from person to person and has different sizes depending on the location.

When the surgical procedure is performed as described above, it is important to delicately locate a component including an electrode to be formed at the end of a catheter so as to enclose the outer wall of the artery. Specifically, in order to effectively denervate or modulate the nerves, the component needs to enclose the outer wall of the artery with distributed nerves in a circumferential direction. Also, it is necessary to reliably and rapidly enclose the artery with the component including the electrode. In particular, it is important to safely and accurately attach the electrode-formed component to the outer wall of the tube in the body so as not to damage the tube in the body, which can be easily damaged by external stimuli.

(Patent Document 1) Korean Patent Laid-open Publication No. 2013-0108401 (published on October 2, 2013)

### DISCLOSURE OF THE INVENTION

### PROBLEMS TO BE SOLVED BY THE INVENTION

The present disclosure is conceived to provide an electrode apparatus capable of identifying a diameter of a tube in the body and performing nerve denervation based on the diameter of the tube in the body.

The problems to be solved by the present disclosure are not limited to the above-described problems.

There may be other problems to be solved by the present disclosure.

### MEANS FOR SOLVING THE PROBLEMS

According to an aspect of the present disclosure, an electrode apparatus for nerve denervation or modulation in vivo includes: a main body including a shaft; and an electrode unit formed to protrude from one end of the shaft, configured to denervate or modulate at least a part of nerves on a tube in a body, and including a base layer and an electrode layer disposed on the base layer. The electrode unit includes: a first electrode disposed along a longitudinal direction of one side of the base layer; an electrode layer including a second electrode disposed along a longitudinal direction of the other side of the base layer; a current sensor unit formed in a region between the first electrode and the second electrode and configured to measure a current generated from the first electrode or the second electrode; a plurality of temperature sensor units spaced apart from each other at a predetermined interval between the first electrode and the second electrode and configured to measure a temperature; and a controller configured to calculate a resistance based on the current measured by the current sensor unit and calculate a diameter of the tube in the body based on the calculated resistance.

The above-described aspects are provided by way of illustration only and should not be construed as liming the present disclosure. Besides the above-described embodiments, there may be additional embodiments described in the accompanying drawings and the detailed description.

### EFFECTS OF THE INVENTION

According to any one of the above-described means for solving the problems of the present disclosure, it is possible to provide an electrode apparatus capable of performing nerve denervation based on a diameter of a tube in the body.

That is, the electrode apparatus includes an electrode unit, and can identify a diameter of a tube in the body and regulate a temperature or a period of time of applying a voltage to an electrode based on the identified diameter of the tube in the body.

### BRIEF DESCRIPTION OF THE DRAWINGS

> **FIG. 1** is a side view of an electrode apparatus according to an embodiment of the present disclosure.
> **FIG. 2** is a plan view of an electrode unit.
> **FIG. 3** illustrates an electrode unit according to an embodiment of the present disclosure.
> **FIG. 4** illustrates an operation process of the electrode unit illustrated in **FIG. 3.**

FIG. 5 illustrates an electrode unit according to another embodiment of the present disclosure.

**FIG. 6A** illustrates an operation process of the electrode unit illustrated in **FIG. 5** according to an embodiment when a diameter of a blood vessel in the body is identified.

**FIG. 6B** illustrates an operation process of the electrode unit illustrated in **FIG. 5** according to an embodiment when a diameter of a blood vessel in the body is not identified.

**FIG. 7** illustrates an operation process of the electrode unit illustrated in **FIG. 5** according to yet another embodiment.

**FIG. 8** is an example diagram provided to explain a thermocouple.

## BEST MODE FOR CARRYING OUT THE INVENTION

Hereinafter, embodiments of the present disclosure will be described in detail with reference to the accompanying drawings to be readily implemented by a person with ordinary skill in the art to which the present invention belongs. However, it is to be noted that the present disclosure is not limited to the example embodiments but can be embodied in various other ways. In the drawings, parts irrelevant to the description are omitted in order to clearly explain the present disclosure, and like reference numerals denote like parts through the whole document.

Through the whole document, the term "connected to" or "coupled to" that is used to designate a connection or coupling of one element to another element includes both a case that an element is "directly connected or coupled to" another element and a case that an element is "electronically connected or coupled to" another element via still another element. Further, it is to be understood that the term "comprises or includes" and/or "comprising or including" used in the document means that one or more other components, steps, operation and/or existence or addition of elements are not excluded in addition to the described components, steps, operation and/or elements unless context dictates otherwise and is not intended to preclude the possibility that one or more other features, numbers, steps, operations, components, parts, or combinations thereof may exist or may be added.

Hereinafter, an embodiment of the present disclosure will be described in detail with reference to the accompanying drawings.

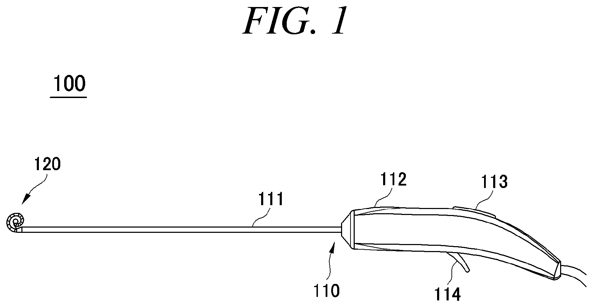

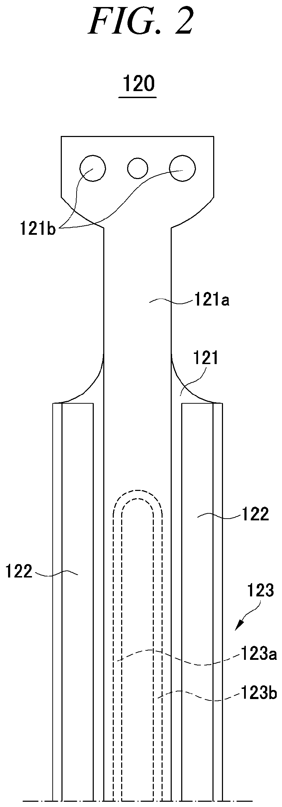

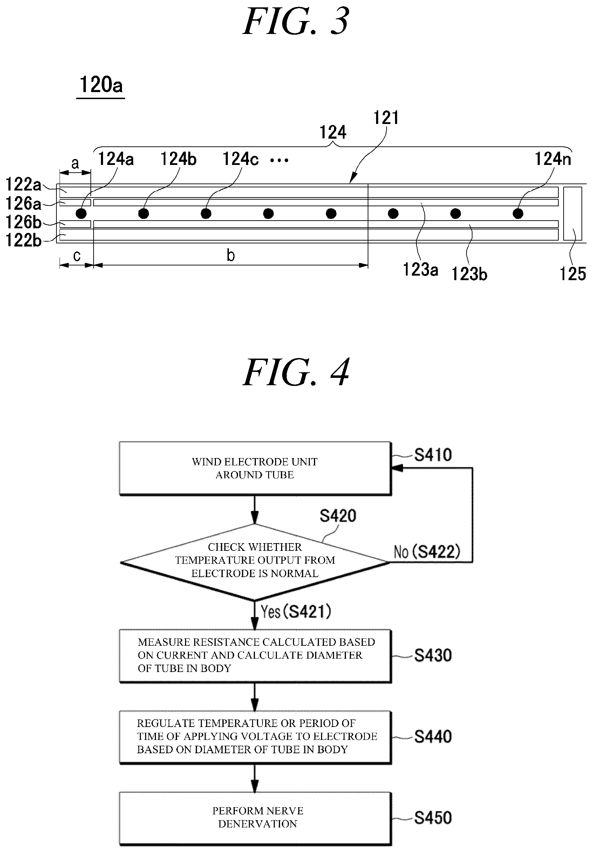

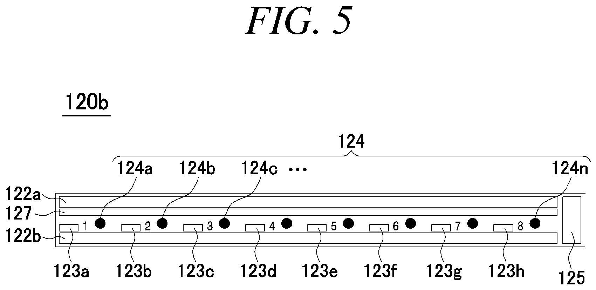

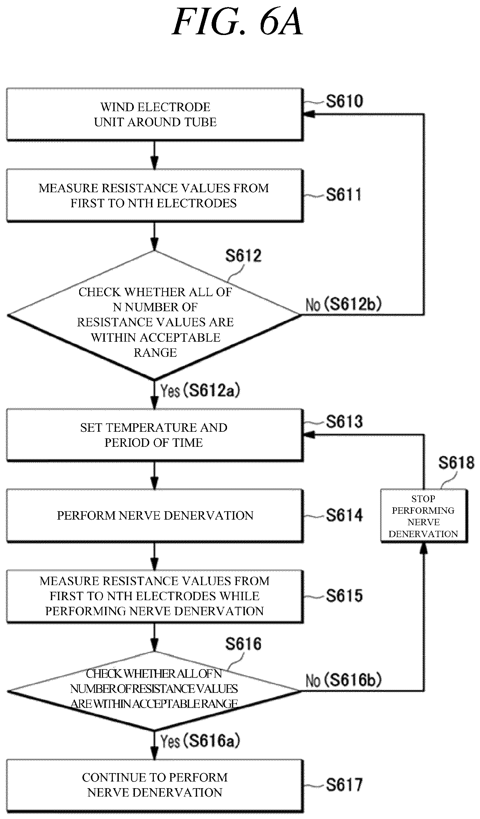

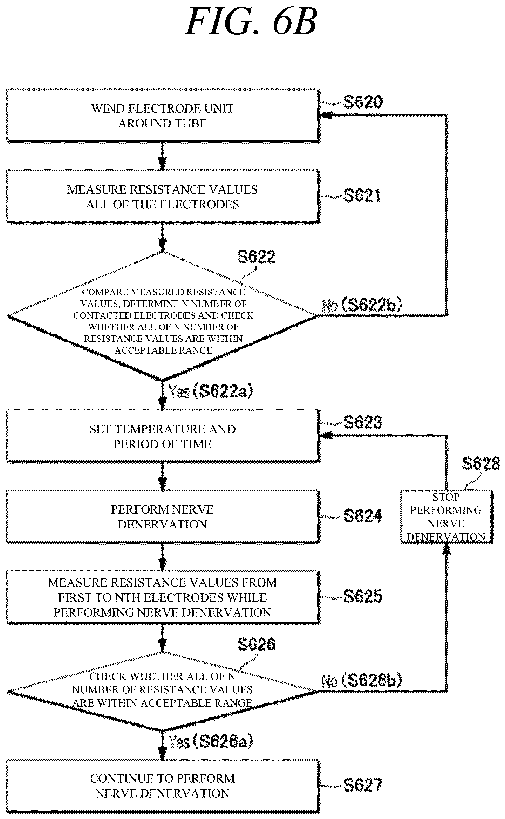

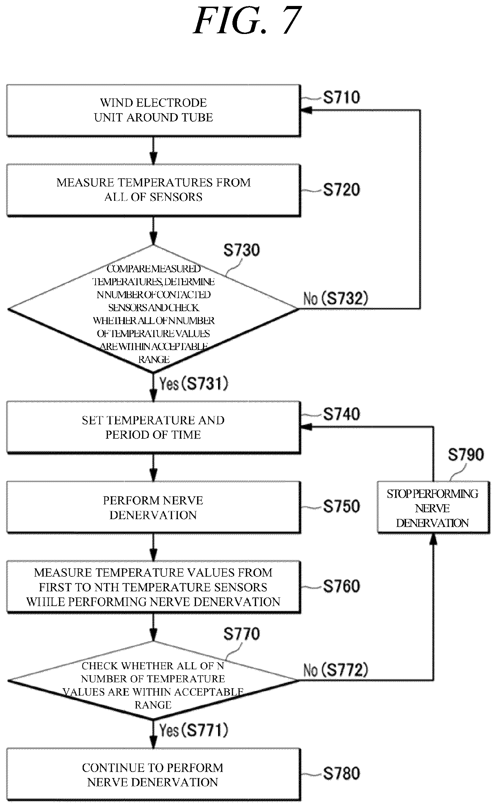

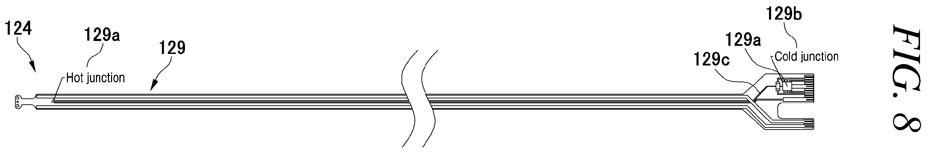

**FIG. 1** is a side view of an electrode apparatus according to an embodiment of the present disclosure, and **FIG. 2** is a plan view of an electrode unit. **FIG. 3** illustrates an electrode unit according to an embodiment of the present disclosure, and **FIG. 4** illustrates an operation process of the electrode unit illustrated in **FIG. 3. FIG. 5** illustrates an electrode unit according to another embodiment of the present disclosure, **FIG. 6A** illustrates an operation process of the electrode unit illustrated in **FIG. 5** according to an embodiment when a diameter of a blood vessel in the body is identified, and **FIG. 6B** illustrates an operation process of the electrode unit illustrated in **FIG. 5** according to an embodiment when a diameter of a blood vessel in the body is not identified. **FIG. 7** illustrates an operation process of the electrode unit illustrated in **FIG. 5** according to yet another embodiment, and **FIG. 8** is an example diagram provided to explain a thermocouple.

Referring to **FIG. 1,** an electrode apparatus 100 includes a main body 110 and an electrode unit 120. The main body 110 may include a shaft 111 extending in one direction, a grip portion 112 connected to the shaft 111 so as to be gripped by an operator, a guide manipulation unit 113 formed on the grip portion 112 so as to manipulate an operation of an electrode guide (not illustrated), and an electrode manipulation unit 114 formed on the grip portion 112 so as to manipulate energy transfer to the electrode unit 120.

The electrode unit 120 is formed to be drawn out from one end of the shaft 111 and configured to denervate or modulate at least a part of nerves distributed on a tissue including a tube in the body depending on manipulation by the operator. The electrode unit 120 is accommodated inside the shaft 111 and when the electrode apparatus 100 operates, the electrode unit 120 can be drawn out.

Throughout the whole document, the term "tube" may include various tubes, such as hepatic arteries, splenic arteries, and pulmonary arteries, and their nerves as well as blood vessels including arteries and vein.

Referring to **FIG. 2**, the electrode unit 120 may include a base layer 121, an electrode layer 122, and a top layer 121a. In the electrode apparatus 100, an electrode encloses an outer surface of a tube or tube-shaped tissue in the body and energy can be transferred through the electrode layer 122. To this end, the base layer 121 may be formed as a flexible printed circuit board (PCB).

The electrode layer 122 may be composed of two electrodes extending parallel to each other on the base layer 121. The base layer 121 and the electrode layer 122 may be configured to extend and enclose the tube in the body or the like in a circumferential direction.

In the embodiment illustrated in **FIG. 2**, the electrode unit 120 may include the base layer 121, the electrode layer 122 disposed on the base layer 121, and the top layer 121a disposed to overlap a part of the electrode layer 122 with the electrode layer 122 interposed therebetween. A through-hole (not illustrated) may be formed in a part of the electrode unit 120.

The electrode layer 122 may be made of a material, such as stainless steel or gold, which is harmless to the human body and conducts electricity well in order to block or denervate or control or modulate the nerves.

Also, the electrode layer 122 may transfer various types of energy from an energy source generator. For example, the energy may include radio-frequency (RF) energy, electrical energy, laser energy, ultrasonic energy, high-intensity focused ultrasound energy, cryogenic energy and other heat energy.

Further, the electrode layer 122 may be implemented as a flexible PCB for transferring RF energy, a transducer for transferring ultrasonic energy or a metal electrode for transferring high-voltage energy and thus may transfer energy to damage the nerves.

Furthermore, a sensor unit 123 may be formed on the base layer 121. For example, the sensor unit 123 may be a thermocouple that measures a temperature by contact with the tube in the body or the like, and when neurotomy is performed with the electrode apparatus 100, the sensor unit 123 may monitor a temperature of a treatment site. As another example, the sensor unit 123 may measure signals from the nerves on the tube.

The sensor unit 123 may be, for example, a thermocouple composed of a first metal 123a and a second metal 123b. For example, the sensor unit 123 may be composed of a pair of copper and constantan.

Referring to **FIG. 3**, an electrode unit 120a according to an embodiment may include a first electrode 122a and a second electrode 122b disposed along a longitudinal direction of the base layer 121. For example, the first electrode 122a may be disposed along the longitudinal direction of one side of the base layer 121, the second electrode 122b may be disposed along the longitudinal direction of the other side of the base layer 121.

The electrode unit 120a according to an embodiment may include a current sensor unit 123, a temperature sensor unit 124, a controller (not illustrated), and a reference current sensor unit 126. Current sensor units 123a and 123b may be formed in a region between the first electrode 122a and the second electrode 122b and configured to measure a current generated from the first electrode 122a or the second electrode 122b. For example, a first current sensor unit 123a may be disposed in the longitudinal direction parallel to the first electrode 122a, and a second current sensor unit 123b may be disposed in the longitudinal direction parallel to the second electrode 122b.

A plurality of temperature sensor units 124 may be spaced apart from each other at a predetermined interval between the first electrode 122a and the second electrode 122b and configured to measure a temperature. For example, the temperature sensor units 124 may be spaced apart from each other at a constant interval between the first electrode 122a and the second electrode 122b. Specifically, the temperature sensor units 124 may include a total of n number of temperature sensors, and a first temperature sensor 124a, a second temperature sensor 124b, a third temperature sensor 124c, and an nth temperature sensor 124n may be spaced spart from each other at a constant interval along the longitudinal direction.

Reference current sensor units 126a and 126b may be formed in the other region between the first electrode 122a and the second electrode 122b. For example, a first reference current sensor unit 126a and a second reference current sensor unit 126b may have a constant length a, and the first reference current sensor unit 126a may be formed at a position parallel to the first electrode 122a and the second reference current sensor unit 126b may be formed at a position parallel to the second electrode 122b.

The reference current sensor units 126a and 126b may apply a constant voltage between the first reference current sensor unit 126a and the second reference current sensor unit 126b and measure a current to calculate a reference resistance. Further, the reference current sensor units 126a and 126b may apply the same voltage to the first current sensor unit 123a and the second current sensor unit 123b and measure a current.

The controller (not illustrated) according to an embodiment may calculate the reference resistance based on the currents measured by the current sensor unit 123 and the reference current sensor unit 126. Specifically, the controller may calculate a diameter of the tube in the body based on a ratio between a length of the current sensor units 123a and 123b and a contact length of the reference current sensor units 126a and 126b with the blood vessel and a ratio between the reference resistance and the resistance (see Equation 1 below).

Herein, the controller may be included in the electrode apparatus 100 or the energy source generator.

<Equation 1>

$$a : b = \Omega_T : \Omega_R$$

$$L = b + c, \quad D = L/\eth$$

Referring to Equation 1 and **FIG. 2**, a is the length of the reference current sensor units 126a and 126b, and b is the length of the current sensor units 123a and 123b in contact with the blood vessel. Also, $\Omega T$ is the resistance of the current sensor units 123a and 123b, and $\Omega R$ is the reference resistance of the reference current sensor units 126a and 126b. Further, L is the contact length of the electrode unit 120 with the tube in the body, and D is the diameter of the tube in the body. Herein, a and c are constants. For example, the controller may compare the resistance $\Omega T$ with the reference resistance $\Omega R$ to calculate the contact length of the electrode unit with the tube in the body. Thus, it is possible to calculate the diameter of the tube.

The controller may regulate a temperature or a period of time of applying a voltage to the first electrode 122a and the second electrode 122b based on the calculated diameter of the tube in the body.

Referring to **FIG. 4**, a process of performing nerve denervation by using the electrode apparatus 100 including the electrode unit 120a will be described. The electrode apparatus 100 according to an embodiment may wind the electrode unit 120a around the tube (S410). The electrode apparatus 100 according to an embodiment may check whether a temperature output from the electrode is normal (S420).

According to the present disclosure, the term "nerve denervation" may refer to renal denervation (RDN).

When the temperature output from the electrode is normal (S421), the electrode apparatus 100 according to an embodiment may measure a resistance calculated based on the current and calculate a diameter of the tube in the body (S430). Meanwhile, when the temperature output from the electrode is not normal (S422), the process may return to the previous operation and the electrode apparatus 100 according to an embodiment wind the electrode unit 120a around the tube (S410).

The electrode apparatus 100 according to an embodiment may regulate a temperature or a period of time of applying a voltage to the electrode based on the diameter of the tube in the body (S440). The electrode apparatus 100 according to an embodiment may perform nerve denervation (S450).

Referring to **FIG. 5**, an electrode unit 120b according to another embodiment may include the first electrode 122a and the second electrode 122b disposed along the longitudinal direction of the base layer 121, and may further include a common line 127 spaced apart from the first electrode 122a at a predetermined distance and disposed along the longitudinal direction parallel to the first electrode 122a. The common line 127 may apply a voltage to current sensor units 128a to 128h.

The electrode unit 120b according to another embodiment may include the current sensor units 128a to 128h, the temperature sensor unit 124, and the controller. As illustrated in **FIG. 5**, the current sensor units 128a to 128h may include a plurality of current sensors spaced apart from each other at a predetermined interval between the first electrode 122a and the second electrode 122b. The temperature sensor unit 124 may include a total of n number of temperature sensors 124a to 124n spaced spart from each other at a constant interval between the first electrode 122a and the second electrode 122b.

The controller may measure a resistance based on currents measured by the current sensor units 128a to 123h. For example, the controller may determine a contact state between the base layer 121 and the tube in the body by comparing resistance values measured by the common line 127 and a first current sensor 128a to an eighth current sensor 128h, respectively.

As another example, the controller may compare the resistance value measured by the fourth current sensor 128d with the resistance values measured by the other current sensors 128a to 128c and 128e to 128h, and may determine that the base layer 121 is in poor contact with the tube in the body when the resistance value measured by the fourth current sensor 128d is lower.

Also, the controller may determine a contact state between the base layer 121 and the tube in the body based on a plurality of temperature values sensed by the temperature sensor unit 124. For example, the controller may determine a contact state between the base layer 121 and the tube in the body by comparing temperature values measured by the first temperature sensor 124a to an nth temperature sensor 124n, respectively.

As another example, the controller may compare temperature values measured by the first temperature sensor 124a to the nth temperature sensor 124n, respectively, and may sense a case where the temperature value measured by the second temperature sensor 124b is much lower or higher. Therefore, the controller can suppress an abnormal contact between the base layer 121 and the tube in the body.

Referring to **FIG. 6A** and **FIG. 6B**, a process of performing nerve denervation by using the electrode apparatus 100 including the electrode unit 120a illustrated in **FIG. 5** according to an embodiment will be described. **FIG. 6A** illustrates a case where a diameter of the blood vessel in the body is identified, and **FIG. 6B** illustrates a case where a diameter of the blood vessel in the body is not identified. Referring to **FIG. 6A** first, the electrode apparatus 100 according to another embodiment may wind the electrode unit 120 around the tube (S610).

The electrode apparatus 100 according to another embodiment may measure resistance values from first to nth electrodes (S611). The electrode apparatus 100 according to another embodiment may check whether all of N number of resistance values are within an acceptable range (S612).

When all of the N number of resistance values are within the acceptable range (S612a), the electrode apparatus 100 according to another embodiment may set a temperature and a period of time (S613). Meanwhile, when all of the N number of resistance values are not within the acceptable range (S612b), the electrode apparatus 100 according to another embodiment may wind the electrode unit 120 around the tube again (S610).

The electrode apparatus 100 according to another embodiment may perform nerve denervation based on the set temperature and period of time (S614). The electrode apparatus 100 according to another embodiment may measure resistance values from the first to nth electrodes while performing nerve denervation (S615).

The electrode apparatus 100 according to another embodiment may check whether all of the N number of resistance values are within the acceptable range (S616). When all of the N number of resistance values are within the acceptable range (S616a), the electrode apparatus 100 according to another embodiment may continue to perform nerve denervation (S617).

When all of the N number of resistance values are not within the acceptable range (S616b), the electrode apparatus 100 according to another embodiment may stop performing nerve denervation (S618). Further, the electrode apparatus 100 may set a temperature and a period of time again (S613).

Referring to **FIG. 6B**, the electrode apparatus 100 including the electrode unit 120a illustrated in **FIG. 5** according to another embodiment may wind the electrode unit 120 around the tube (S620). The electrode apparatus 100 according to another embodiment may measure resistance values from all of the electrodes (S621).

The electrode apparatus 100 according to another embodiment may compare the measured resistance values, determine N number of contacted electrodes and check whether all of N number of resistance values are within the acceptable range (S622).

When all of the N number of resistance values are within the acceptable range (S622a), the electrode apparatus 100 according to another embodiment may set a temperature and a period of time (S623). Meanwhile, when all of the N number of resistance values are not within the acceptable range (S622b), the electrode apparatus 100 according to another embodiment may wind the electrode unit 120 around the tube again (S620).

The electrode apparatus 100 according to another embodiment may perform nerve denervation based on the set temperature and period of time (S624).

The electrode apparatus 100 according to another embodiment may measure resistance values from the first to nth electrodes while performing nerve denervation (S625). The electrode apparatus 100 according to another embodiment may check whether all of the N number of resistance values are within the acceptable range (S626).

When all of the N number of resistance values are within the acceptable range (S626a), the electrode apparatus 100 according to another embodiment may continue to perform nerve denervation (S627). When all of the N number of resistance values are not within the acceptable range (S626b), the electrode apparatus 100 according to another embodiment may stop performing nerve denervation (S628). Further, the electrode apparatus 100 may set a temperature and a period of time again (S623).

Referring to **FIG. 7**, a process of performing nerve denervation by using the electrode apparatus 100 including the electrode unit 120a illustrated in **FIG. 5** according to yet another embodiment will be described.

Referring to 7, the electrode apparatus 100 including the electrode unit 120a according to yet another embodiment may wind the electrode unit 120 around the tube (S710). The electrode apparatus 100 according to yet another embodiment may measure temperatures from all of sensors (S720).

The electrode apparatus 100 according to yet another embodiment may compare the measured temperatures, determine N number of contacted sensors and check whether all of N number of temperature values are within an acceptable range (S730).

When all of the N number of temperature values are within the acceptable range (S731), the electrode apparatus 100 according to yet another embodiment may set a temperature and a period of time (S740). Meanwhile, when all of the N number of temperature values are not within the acceptable range (S732), the electrode apparatus 100 according to yet another embodiment may wind the electrode unit 120 around the tube again (S710).

The electrode apparatus 100 according to yet another embodiment may perform nerve denervation based on the set temperature and period of time (S750). The electrode apparatus 100 according to yet another embodiment may measure temperature values from first to nth temperature sensors while performing nerve denervation (S760).

The electrode apparatus 100 according to yet another embodiment may check whether all of the N number of temperature values are within the acceptable range (S770). When all of the N number of temperature values are within the acceptable range (S771), the electrode apparatus 100 according to yet another embodiment may continue to perform nerve denervation (S780).

Meanwhile, when all of the N number of temperature values are not within the acceptable range (S772), the electrode apparatus 100 according to yet another embodiment may stop performing nerve denervation (S790). Further, the electrode apparatus 100 may set a temperature and a period of time again (S740).

Referring to **FIG. 8**, the temperature sensor unit 124 may further include a thermocouple 129 to improve the accuracy in measuring a temperature. For example, the temperature sensor unit 124 may include at least one temperature chip and the thermocouple 129.

As illustrated in **FIG. 8**, the thermocouple 129 is of T-type including copper (not illustrated) and a constantan 129c.

For example, one side of the thermocouple 129 may be a hot junction area 129a, and the other side may be a cold junction area 129b. The hot junction area 129a refers to an area in direct contact with the tube in the body. In order to accurately measure a temperature of the hot junction area 129a, a temperature of the cold junction area 129b needs to be detected.

The cold junction area 129b of the thermocouple 129 may be an area where the constantan 129c is in contact with the copper. The thermocouple 129 may include a temperature chip 129d in the cold junction area 129b to detect the temperature of the cold junction area 129b.

The thermocouple 129 may be configured in various ways, such as J-type, K-type, T-type, E-type, N-type, R-type, S-type, etc.

The above description of the present disclosure is provided for the purpose of illustration, and it would be understood by a person with ordinary skill in the art to which the present disclosure belongs that various changes and modifications may be made without changing technical conception and essential features of the present disclosure. Thus, it is clear that the above-described examples are illustrative in all aspects and do not limit the present disclosure. For example, each component described to be of a single type can be implemented in a distributed manner, likewise, components described to be distributed can be implemented in a combined manner.

The scope of the present disclosure is defined by the following claims rather than by the detailed description of the embodiment, and it should be understood that all modifications and embodiments conceived from the meaning and scope of the claims and their equivalents are included in the scope of the present disclosure.

**Claims**

1. An electrode apparatus for nerve denervation or modulation in vivo, comprising:

   a main body including a shaft; and
   an electrode unit formed to protrude from one end of the shaft, configured to denervate or modulate at least a part of nerves on a tube in a body, and including a base layer and an electrode layer disposed on the base layer,
   wherein the electrode unit includes:

   a first electrode disposed along a longitudinal direction of one side of the base layer;
   an electrode layer including a second electrode disposed along a longitudinal direction of the other side of the base layer;
   a current sensor unit formed in a region between the first electrode and the second electrode and configured to measure a current generated from the first electrode or the second electrode;
   a plurality of temperature sensor units spaced apart from each other at a predetermined interval between the first electrode and the second electrode and configured to measure a temperature; and
   a controller configured to calculate a resistance based on the current measured by the current sensor unit and calculate a diameter of the tube in the body based on the calculated resistance.

2. The electrode apparatus of Claim 1, wherein the controller regulates a temperature or a period of time of applying a voltage to the first electrode and the second electrode based on the calculated diameter of the tube in the body.

3. The electrode apparatus of Claim 1, further comprising:
   a reference current sensor unit formed in the other region between the first electrode and the second electrode and configured to measure currents generated from the first electrode and the second electrode in order to calculate a reference resistance.

4. The electrode apparatus of Claim 3, wherein the controller calculates the reference resistance based on the currents measured by the reference current sensor unit, and calculates the diameter of the tube in the body based on a ratio between a length of the current sensor unit and a length of the reference current sensor unit and a ratio between the reference resistance and the resistance.

5. The electrode apparatus of Claim 1, further comprising:
   a common line spaced apart from the first electrode at a predetermined distance, disposed along the longitudinal direction parallel to the first electrode, and configured to apply a voltage to the first electrode.

6. The electrode apparatus of Claim 1, wherein the controller determines a contact state between the base layer and the tube in the body based on a plurality of temperature values sensed by the temperature sensor units.

7. The electrode apparatus of Claim 1,

   wherein each of the temperature sensor units is configured as a thermocouple, and
   the thermocouple includes a temperature chip in a cold junction.

# FIG. 1

<u>100</u>

## FIG. 2

<u>120</u>

# FIG. 3

<u>120a</u>

# FIG. 4

```
                    ┌─────────────────────────┐
                    │   WIND ELECTRODE UNIT    │──── S410
                    │      AROUND TUBE         │
                    └─────────────────────────┘
                              │
                              ▼
                         ╱ S420 ╲
                    ╱  CHECK WHETHER  ╲
                  ╱  TEMPERATURE OUTPUT FROM ╲─── No (S422)
                  ╲   ELECTRODE IS NORMAL   ╱
                    ╲                     ╱
                              │
                          Yes (S421)
                              ▼
        ┌──────────────────────────────────────┐
        │ MEASURE RESISTANCE CALCULATED BASED   │── S430
        │ ON CURRENT AND CALCULATE DIAMETER     │
        │ OF TUBE IN BODY                       │
        └──────────────────────────────────────┘
                              │
                              ▼
        ┌──────────────────────────────────────┐
        │ REGULATE TEMPERATURE OR PERIOD OF     │── S440
        │ TIME OF APPLYING VOLTAGE TO ELECTRODE │
        │ BASED ON DIAMETER OF TUBE IN BODY     │
        └──────────────────────────────────────┘
                              │
                              ▼
        ┌──────────────────────────────────────┐
        │        PERFORM NERVE                  │── S450
        │        DENERVATION                    │
        └──────────────────────────────────────┘
```

# *FIG. 5*

<u>120b</u>

# FIG. 6A

```
┌─────────────────────────┐
│   WIND ELECTRODE        │  ~S610
│   UNIT AROUND TUBE      │
└─────────────────────────┘
            │
            ▼
┌─────────────────────────┐
│ MEASURE RESISTANCE      │  ~S611
│ VALUES FROM FIRST TO    │
│ NTH ELECTRODES          │
└─────────────────────────┘
            │
            ▼
        S612
    ╱ CHECK WHETHER ALL OF ╲     No (S612b)
   ╱   N NUMBER OF          ╲
  ╱  RESISTANCE VALUES ARE   ╲
  ╲  WITHIN ACCEPTABLE       ╱
   ╲     RANGE              ╱
    ╲                     ╱
      Yes (S612a)
            │
            ▼
┌─────────────────────────┐
│ SET TEMPERATURE AND     │  ~S613
│ PERIOD OF TIME          │
└─────────────────────────┘
            │                        S618
            ▼              ┌──────────────────┐
┌─────────────────────────┐│ STOP PERFORMING  │
│ PERFORM NERVE           ││ NERVE            │
│ DENERVATION             ││ DENERVATION      │  ~S614
└─────────────────────────┘└──────────────────┘
            │
            ▼
┌─────────────────────────┐
│ MEASURE RESISTANCE      │  ~S615
│ VALUES FROM FIRST TO    │
│ NTH ELECTRODES WHILE    │
│ PERFORMING NERVE        │
│ DENERVATION             │
└─────────────────────────┘
            │
            ▼
        S616
    ╱ CHECK WHETHER ALL OF N ╲    No (S616b)
   ╱ NUMBER OF RESISTANCE VALUES ╲
   ╲ ARE WITHIN ACCEPTABLE RANGE ╱
      Yes (S616a)
            │
            ▼
┌─────────────────────────┐
│ CONTINUE TO PERFORM     │  ~S617
│ NERVE DENERVATION       │
└─────────────────────────┘
```

# FIG. 6B

WIND ELECTRODE UNIT AROUND TUBE — S620

MEASURE RESISTANCE VALUES ALL OF THE ELECTRODES — S621

S622

COMPARE MEASURED RESISTANCE VALUES, DETERMINE N NUMBER OF CONTACTED ELECTRODES AND CHECK WHETHER ALL OF N NUMBER OF RESISTANCE VALUES ARE WITHIN ACCEPTABLE RANGE

No (S622b)

Yes (S622a)

SET TEMPERATURE AND PERIOD OF TIME — S623

S628

STOP PERFORMING NERVE DENERVATION

PERFORM NERVE DENERVATION — S624

MEASURE RESISTANCE VALUES FROM FIRST TO NTH ELECTRODES WHILE PERFORMING NERVE DENERVATION — S625

S626

CHECK WHETHER ALL OF N NUMBER OF RESISTANCE VALUES ARE WITHIN ACCEPTABLE RANGE

No (S626b)

Yes (S626a)

CONTINUE TO PERFORM NERVE DENERVATION — S627

# FIG. 7

```
┌─────────────────────────┐
│   WIND ELECTRODE        │──── S710
│   UNIT AROUND TUBE      │
└─────────────────────────┘
            │
            ▼
┌─────────────────────────┐
│ MEASURE TEMPERATURES    │──── S720
│ FROM ALL OF SENSORS     │
└─────────────────────────┘
            │
            ▼
        ◇ S730
   COMPARE MEASURED
   TEMPERATURES, DETERMINE
   N NUMBER OF CONTACTED
   SENSORS AND CHECK          No (S732)
   WHETHER ALL OF N NUMBER
   OF TEMPERATURE VALUES
   ARE WITHIN ACCEPTABLE
   RANGE
            │
        Yes (S731)
            ▼
┌─────────────────────────┐
│ SET TEMPERATURE AND     │──── S740
│ PERIOD OF TIME          │
└─────────────────────────┘
            │
            ▼                     S790
┌─────────────────────────┐   ┌──────────────┐
│ PERFORM NERVE           │── │ STOP         │
│ DENERVATION             │S750│ PERFORMING   │
└─────────────────────────┘   │ NERVE        │
            │                 │ DENERVATION  │
            ▼                 └──────────────┘
┌─────────────────────────┐
│ MEASURE TEMPERATURE     │──── S760
│ VALUES FROM FIRST TO    │
│ NTH TEMPERATURE SENSORS │
│ WHILE PERFORMING NERVE  │
│ DENERVATION             │
└─────────────────────────┘
            │
            ▼
        ◇ S770
   CHECK WHETHER ALL OF N
   NUMBER OF TEMPERATURE      No (S772)
   VALUES ARE WITHIN ACCEPTABLE
   RANGE
            │
        Yes (S771)
            ▼
┌─────────────────────────┐
│ CONTINUE TO PERFORM     │──── S780
│ NERVE DENERVATION       │
└─────────────────────────┘
```

## FIG. 8

## INTERNATIONAL SEARCH REPORT

| | International application No. |
|---|---|
| | **PCT/KR2022/019470** |

### A. CLASSIFICATION OF SUBJECT MATTER

**A61B 18/14**(2006.01)i; **A61B 18/20**(2006.01)i; **A61B 18/02**(2006.01)i; **A61N 1/40**(2006.01)i; **A61N 1/06**(2006.01)i; **A61N 1/05**(2006.01)i; **A61N 1/36**(2006.01)i; **A61N 7/02**(2006.01)i

According to International Patent Classification (IPC) or to both national classification and IPC

### B. FIELDS SEARCHED

Minimum documentation searched (classification system followed by classification symbols)

A61B 18/14(2006.01); A61B 18/00(2006.01); A61B 5/053(2006.01); A61B 5/107(2006.01); A61N 1/36(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 신경(nerve), 차단(blocking), 자극(stimulate), 전극(electrode), 기준(reference), 온도 (temperature), 전류(current), 센서(sensor)

### C. DOCUMENTS CONSIDERED TO BE RELEVANT

| Category* | Citation of document, with indication, where appropriate, of the relevant passages | Relevant to claim No. |
|---|---|---|
| Y | KR 10-2347531 B1 (DEEPQURE INC.) 07 January 2022 (2022-01-07)<br>See paragraphs [0036]-[0055]. | 1-7 |
| Y | JP 2021-516595 A (MEDTRONIC ARDIAN LUXEMBOURG S.A.R.L.) 08 July 2021 (2021-07-08)<br>See paragraphs [0022] and [0052]. | 1-7 |
| Y | US 2020-0129087 A1 (FOUNDRY INNOVATION & RESEARCH 1, LTD.) 30 April 2020 (2020-04-30)<br>See paragraph [0173]. | 3,4 |
| Y | JP 2015-188550 A (OLYMPUS CORP.) 02 November 2015 (2015-11-02)<br>See paragraphs [0164] and [0170]. | 6,7 |
| A | KR 10-2018-0103385 A (KOREA ADVANCED INSTITUTE OF SCIENCE AND TECHNOLOGY) 19 September 2018 (2018-09-19)<br>See entire document. | 1-7 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **25 January 2024** | **25 January 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT
Information on patent family members

International application No.

**PCT/KR2022/019470**

| Patent document cited in search report | | | Publication date (day/month/year) | Patent family member(s) | | | Publication date (day/month/year) |
|---|---|---|---|---|---|---|---|
| KR | 10-2347531 | B1 | 07 January 2022 | WO | 2022-260203 | A1 | 15 December 2022 |
| JP | 2021-516595 | A | 08 July 2021 | CN | 111867508 | A | 30 October 2020 |
| | | | | EP | 3742999 | A1 | 02 December 2020 |
| | | | | JP | 7250818 | B2 | 03 April 2023 |
| | | | | US | 2019-0223754 | A1 | 25 July 2019 |
| | | | | WO | 2019-147474 | A1 | 01 August 2019 |
| US | 2020-0129087 | A1 | 30 April 2020 | EP | 3629921 | A1 | 08 April 2020 |
| | | | | US | 11779238 | B2 | 10 October 2023 |
| | | | | US | 2023-0414124 | A1 | 28 December 2023 |
| | | | | WO | 2018-220146 | A1 | 06 December 2018 |
| JP | 2015-188550 | A | 02 November 2015 | | None | | |
| KR | 10-2018-0103385 | A | 19 September 2018 | KR | 10-2022761 | B1 | 19 September 2019 |

Form PCT/ISA/210 (patent family annex) (July 2022)

**REFERENCES CITED IN THE DESCRIPTION**

**Patent documents cited in the description**

- KR 20130108401